# EUROPEAN PATENT APPLICATION

(11) **EP 4 393 945 A1**
(43) Date of publication of application: **03.07.2024**
(21) Application number: 22860599.4
(22) Date of filing: 25.08.2022
(51) Int. Cl.: C07K 16/10, C12N 15/13, G01N 33/569, A61K 39/42, A61P 31/14

(54) **ANTI-RSV ANTIBODY AND APPLICATION THEREOF**

(30) Priority: 25.08.2021 CN 202110978489
(71) Applicant: GAN & LEE PHARMACEUTICALS CO., LTD., Beijing 101109 (CN)
(72) Inventor: JIAO, Jiao, Beijing 101109 (CN); QIAO, Jing, Beijing 101109 (CN); LI, Ruowei, Beijing 101109 (CN); LIU, Yaohui, Beijing 101109 (CN); WANG, Jiaxing, Beijing 101109 (CN); WANG, Zhen, Beijing 101109 (CN); QIN, Han, Beijing 101109 (CN); MA, Ziyue, Beijing 101109 (CN); WANG, Jiao, Beijing 101109 (CN); ZHANG, Ling, Beijing 101109 (CN); CHE, Liming, Beijing 101109 (CN); TIAN, Shumiao, Beijing 101109 (CN); WANG, Ruixue, Beijing 101109 (CN); YAN, Weihuan, Beijing 101109 (CN); JIANG, Yating, Beijing 101109 (CN)
(74) Representative: Kraus & Lederer PartGmbB
(86) International application number: PCT/CN2022/114867
(87) International publication number: WO 2023/025253

(57) **Abstract**

Provided are an antibody having effective neutralizing activity against RSV or an antigen-binding fragment thereof. The provided anti-RSV antibody or antigen-binding fragment thereof can be used as a drug for treating or preventing RSV infection or symptoms related to RSV infection. The provided RSV antibody, after humanization, can bind RSV-F protein with high affinity. In addition, experiments show that it is significantly better than palivizumab at providing protection against RSV challenge.

## Description

### Technical Field

The present invention relates to anti-RSV antibodies, methods of preparing the antibodies, pharmaceutical compositions comprising the antibodies, and the use of the antibodies in the preparation of pharmaceuticals for use in the treatment and/or prevention of human respiratory syncytial virus (RSV) infections or RSV-related symptoms.

### Background Art

Human respiratory syncytial virus (RSV), an antisense, single-stranded RNA virus, is one of the most common viral pathogens causing respiratory disease in infants, young children, the elderly, and immunocompromised adults. RSV is the leading cause of lower respiratory tract infections in infants and small children, and it is the leading cause of hospitalization for respiratory disease in young children. With nearly all children experiencing one or more infections by the age of 4 years, and the peak age of infection is 2 to 8 months. Multiple studies have shown that severe infections in infants are a high risk factor for later asthma, much more dangerous than other microbial pathogens. RSV can also infect adult populations, where it is the main cause of upper respiratory disease, and elder patients and immunocompromised adults, especially bone marrow transplant recipients, may be at greater risk for severe infections and pneumonia.

Natural infection with RSV does not produce sufficient immunity to generate lasting immunity; therefore, the distinguishing feature of RSV infection is that antibodies produced in the body from previous infections do not provide permanent protection, and re-infection can be caused by different subtypes of RSV during the same epidemic season, and even the occurrence of multiple natural infections with RSV does not induce lifelong immune protection of the upper respiratory tract against viral infections, therefore superinfections are common.

Currently, several approaches to preventing and treating RSV infection have been investigated, including vaccine development, antiviral compounds (Ribavirin), antisense drugs, RNA interference techniques, and antibody products. Ribavirin is a nucleoside antimetabolite with severe toxicity and teratogenic effects; although a vaccine may be useful, several vaccine candidates have been discarded, others are in development, and to date there are no vaccine products that have been marketed; two glycoproteins on the surface of RSV, F and G, have been demonstrated to be the target of neutralizing antibodies. Palivizumab, which is specific to the antigenic determining region of RSV-F protein, has been approved for administration to pediatric patients at a monthly dose of 15 mg/kg throughout the RSV season (November-April in the northern hemisphere) for the prevention of severe lower respiratory tract disease caused by RSV; however, palivizumab is an expensive, humanized monoclonal antibody and can only be used for prophylactic treatment of pediatric patients. Therefore, there is an urgent need to develop new anti-RSV drugs, especially those that can treat and/or prevent RSV infection in a wider population.

### Summary

In order to solve the problems in the prior art, the purpose of the present invention is to provide an antibody against respiratory syncytial virus and its application.

The present invention successfully obtains RSV-specific antibody with comparable or better characteristics than previous RSV-specific antibody, these characteristics comprising attacks against RSV A subtypes and/or RSV B subtypes, affinity for RSV, IC₅₀ value and animal efficacy results, etc.

In a first aspect, the present invention provides an antibody or antigen-binding fragment thereof that specifically binds to the respiratory syncytial virus fusion protein (RSV-F) and/or neutralizes respiratory syncytial virus, as determined by surface plasmon resonance, the antibody or antigen-binding fragment thereof specifically binds to RSV-F with an equilibrium dissociation constant (K_{D}) no higher than 10⁻⁹ M.

Optionally, an antibody or antigen-binding fragment thereof, which specifically binds to respiratory syncytial virus fusion protein (RSV-F) and/or neutralizes respiratory syncytial virus, the antibody or antigen-binding fragment thereof comprise:
three heavy chain complementarity determining regions (CDRs) (HCDR1, HCDR2 and HCDR3) contained within the amino acid sequence SEQ ID NO: 1, 3, 5, 7, 9, 11, 13, 15 or 17; and/or
three light chain complementarity determining regions (CDRs) (LCDR1, LCDR2 and LCDR3) contained within the amino acid sequence SEQ ID NO: 2, 4, 6, 8, 10, 12, 14, 16, 18, 29, 30, 31, 32, 33, 34, 35 or 36

Optionally, an antibody or antigen-binding fragment thereof, which specifically binds to respiratory syncytial virus fusion protein (RSV-F) and/or neutralizes respiratory syncytial virus, the antibody or antigen-binding fragment thereof comprises:
a) a HCDR3 functional region, the HCDR3 functional region has any one amino acid sequence selected from the group consisting of the following amino acid sequences: SEQ ID NO: 39, 45, 51, 57, 63, 69, 75, 81, and 87; and
b) a LCDR3 functional region, the HCDR3 functional region has any one amino acid sequence selected from the group consisting of the following amino acid sequences: SEQ ID NO: 42, 48, 54, 60, 66, 72, 78, 84, and 90. Optionally, further comprising:
c) a HCDR1 functional region, the HCDR1 functional region has any one amino acid sequence selected from the group consisting of the following amino acid sequences: SEQ ID NO: 37, 43, 49, 55, 61, 67, 73, 79, and 85;
d) a LCDR1 functional region, the LCDR1 functional region has any one amino acid sequence selected from the group consisting of the following amino acid sequences: SEQ ID NO: 40, 46, 52, 58, 64, 70, 73, 79, 85, 109, 110, 111, 112, 113, 114, 115 and 116;
e) a HCDR2 functional region, the HCDR2 functional region has an amino acid sequence optionally selected from the group consisting of the following amino acid sequences: SEQ ID NO: 38, 44, 50, 56, 62, 68, 74, 80, and 86; and
f) a LCDR2 functional region, the LCDR2 functional region has an amino acid sequence optionally selected from the group consisting of the following amino acid sequences: SEQ ID NO: 41, 47, 53, 59, 65, 71, 77, 83, and 89.

Optionally, the antibody or antigen-binding fragment thereof comprising:
1) three heavy chain complementarity determining regions (CDRs) (HCDR1, HCDR2 and HCDR3) contained within the amino acid sequence SEQ ID NO: 13, and
   three light chain complementarity determining regions (CDRs) (LCDR1, LCDR2 and LCDR3) contained within the amino acid sequence SEQ ID NO: 14, 29, 30, 31, 32, 33, 34, 35, or 36; or
2) three heavy chain complementarity determining regions (CDRs) (HCDR1, HCDR2 and HCDR3) contained within the amino acid sequence SEQ ID NO:1, and
   three light chain complementarity determining regions (CDRs) (LCDR1, LCDR2 and LCDR3) contained within the amino acid sequence SEQ ID NO:2; or
3) three heavy chain complementarity determining regions (CDRs) (HCDR1, HCDR2 and HCDR3) contained within the amino acid sequence SEQ ID NO:3, and
   three light chain complementarity determining regions (CDRs) (LCDR1, LCDR2 and LCDR3) contained within the amino acid sequence SEQ ID NO:4; or
4) three heavy chain complementarity determining regions (CDRs) (HCDR1, HCDR2 and HCDR3) contained within the amino acid sequence SEQ ID NO: 5, and
   three light chain complementarity determining regions (CDRs) (LCDR1, LCDR2 and LCDR3) contained within the amino acid sequence SEQ ID NO:6; or
5) three heavy chain complementarity determining regions (CDRs) (HCDR1, HCDR2 and HCDR3) contained within the amino acid sequence SEQ ID NO:7, and
   three light chain complementarity determining regions (CDRs) (LCDR1, LCDR2 and LCDR3) contained within the amino acid sequence SEQ ID NO:8; or
6) three heavy chain complementarity determining regions (CDRs) (HCDR1, HCDR2 and HCDR3) contained within the amino acid sequence SEQ ID NO:9, and
   three light chain complementarity determining regions (CDRs) (LCDR1, LCDR2 and LCDR3) contained within the amino acid sequence SEQ ID NO: 10; or
7) three heavy chain complementarity determining regions (CDRs) (HCDR1, HCDR2 and HCDR3) contained within the amino acid sequence SEQ ID NO: 11, and
   three light chain complementarity determining regions (CDRs) (LCDR1, LCDR2 and LCDR3) contained within the amino acid sequence SEQ ID NO: 12; or
8) three heavy chain complementarity determining regions (CDRs) (HCDR1, HCDR2 and HCDR3) contained within the amino acid sequence SEQ ID NO: 15, and
   three light chain complementarity determining regions (CDRs) (LCDR1, LCDR2 and LCDR3) contained within the amino acid sequence SEQ ID NO: 16; or
9) three heavy chain complementarity determining regions (CDRs) (HCDR1, HCDR2 and HCDR3) contained within the amino acid sequence SEQ ID NO: 17, and
   three light chain complementarity determining regions (CDRs) (LCDR1, LCDR2 and LCDR3) contained within the amino acid sequence SEQ ID NO: 18.

Optionally, the antibody or antigen-binding fragment thereof comprising:
1) comprising a HCDRlshown in SEQ ID NO:37,
   comprising a HCDR2 shown in SEQ ID NO:38,
   comprising a HCDR3 shown in SEQ ID NO:39,
   comprising a LCDR1 shown in SEQ ID NO:40,
   comprising a LCDR2 shown in SEQ ID NO:41, and
   comprising a LCDR3 shown in SEQ ID NO:42; or
2) comprising a HCDR1 shown in SEQ ID NO:43,
   comprising a HCDR2 shown in SEQ ID NO:44,
   comprising a HCDR3 shown in SEQ ID NO:45,
   comprising a LCDR1 shown in SEQ ID NO:46,
   comprising a LCDR2 shown in SEQ ID NO:47, and
   comprising a LCDR3 shown in SEQ ID NO:48; or
3) comprising a HCDR1 shown in SEQ ID NO:49,
   comprising a HCDR2 shown in SEQ ID NO:50,
   comprising a HCDR3 shown in SEQ ID NO:51,
   comprising a LCDR1 shown in SEQ ID NO:52,
   comprising a LCDR2 shown in SEQ ID NO:53, and
   comprising a LCDR3 shown in SEQ ID NO:54; or
4) comprising a HCDR1 shown in SEQ ID NO:55,
   comprising a HCDR2 shown in SEQ ID NO:56,
   comprising a HCDR3 shown in SEQ ID NO:57,
   comprising a LCDR1 shown in SEQ ID NO:58,
   comprising a LCDR2 shown in SEQ ID NO:59, and
   comprising a LCDR3 shown in SEQ ID NO:60; or
5) comprising a HCDR1 shown in SEQ ID NO:61,
   comprising a HCDR2 shown in SEQ ID NO:62,
   comprising a HCDR3 shown in SEQ ID NO:63,
   comprising a LCDR1 shown in SEQ ID NO:64,
   comprising a LCDR2 shown in SEQ ID NO:65, and
   comprising a LCDR3 shown in SEQ ID NO:66; or
6) comprising a HCDR1 shown in SEQ ID NO:67,
   comprising a HCDR2 shown in SEQ ID NO:68,
   comprising a HCDR3 shown in SEQ ID NO:69,
   comprising a LCDR1 shown in SEQ ID NO:70,
   comprising a LCDR2 shown in SEQ ID NO:71, and
   comprising a LCDR3 shown in SEQ ID NO:72; or
7) comprising a HCDR1 shown in SEQ ID NO:73,
   comprising a HCDR2 shown in SEQ ID NO:74,
   comprising a HCDR3 shown in SEQ ID NO:75,
   comprising a LCDR1 shown in SEQ ID NO:76,
   comprising a LCDR2 shown in SEQ ID NO:77, and
   comprising a LCDR3 shown in SEQ ID NO:78; or
8) comprising a HCDR1 shown in SEQ ID NO:79,
   comprising a HCDR2 shown in SEQ ID NO:80,
   comprising a HCDR3 shown in SEQ ID NO:81,
   comprising a LCDR1 shown in SEQ ID NO:82,
   comprising a LCDR2 shown in SEQ ID NO:83, and
   comprising a LCDR3 shown in SEQ ID NO:84; or
9) comprising a HCDR1 shown in SEQ ID NO:85,
   comprising a HCDR2 shown in SEQ ID NO:86,
   comprising a HCDR3 shown in SEQ ID NO:87,
   comprising a LCDR1 shown in SEQ ID NO:88,
   comprising a LCDR2 shown in SEQ ID NO:89, and
   comprising a LCDR3 shown in SEQ ID NO:90; or
10) comprising a HCDR1 shown in SEQ ID NO:73,
   comprising a HCDR2 shown in SEQ ID NO:74,
   comprising a HCDR3 shown in SEQ ID NO:75,
   comprising a LCDR1 shown in SEQ ID NO:109,
   comprising a LCDR2 shown in SEQ ID NO:77, and
   comprising a LCDR3 shown in SEQ ID NO:78; or
11) comprising a HCDR1 shown in SEQ ID NO:73,
   comprising a HCDR2 shown in SEQ ID NO:74,
   comprising a HCDR3 shown in SEQ ID NO:75,
   comprising a LCDR1 shown in SEQ ID NO:110,
   comprising a LCDR2 shown in SEQ ID NO:77, and
   comprising a LCDR3 shown in SEQ ID NO:78; or
12) comprising a HCDR1 shown in SEQ ID NO:73,
   comprising a HCDR2 shown in SEQ ID NO:74,
   comprising a HCDR3 shown in SEQ ID NO:75,
   comprising a LCDR1 shown in SEQ ID NO:111,
   comprising a LCDR2 shown in SEQ ID NO:77, and
   comprising a LCDR3 shown in SEQ ID NO:78; or
13) comprising a HCDR1 shown in SEQ ID NO:73,
   comprising a HCDR2 shown in SEQ ID NO:74,
   comprising a HCDR3 shown in SEQ ID NO:75,
   comprising a LCDR1 shown in SEQ ID NO:112,
   comprising a LCDR2 shown in SEQ ID NO:77, and
   comprising a LCDR3 shown in SEQ ID NO:78; or
14) comprising a HCDR1 shown in SEQ ID NO:73,
   comprising a HCDR2 shown in SEQ ID NO:74,
   comprising a HCDR3 shown in SEQ ID NO:75,
   comprising a LCDR1 shown in SEQ ID NO: 113,
   comprising a LCDR2 shown in SEQ ID NO:77, and
   comprising a LCDR3 shown in SEQ ID NO:78; or
15) comprising a HCDR1 as shown in SEQ ID NO:73,
   comprising a HCDR2 shown in SEQ ID NO:74,
   comprising a HCDR3 shown in SEQ ID NO:75,
   comprising a LCDR1 shown in SEQ ID NO:114,
   comprising a LCDR2 shown in SEQ ID NO:77, and
   comprising a LCDR3 shown in SEQ ID NO:78; or
16) comprising a HCDR1 shown in SEQ ID NO:73,
   comprising a HCDR2 shown in SEQ ID NO:74,
   comprising a HCDR3 shown in SEQ ID NO:75,
   comprising a LCDR1 shown in SEQ ID NO: 115,
   comprising a LCDR2 shown in SEQ ID NO:77, and
   comprising a LCDR3 shown in SEQ ID NO:78; or
17) comprising a HCDR1 shown in SEQ ID NO:73,
   comprising a HCDR2 shown in SEQ ID NO:74,
   comprising a HCDR3 shown in SEQ ID NO:75,
   comprising a LCDR1 shown in SEQ ID NO: 116,
   comprising a LCDR2 shown in SEQ ID NO:77, and
   comprising a LCDR3 shown in SEQ ID NO:78.

The present invention also discloses an antibody or an antigen-binding fragment thereof, which specifically binds to respiratory syncytial virus fusion protein (RSV-F) and/or neutralizes respiratory syncytial virus, the antibody or antigen-binding fragment thereof comprises:
a heavy chain variable region, the heavy chain variable region has at least 80% identity with any one amino acid sequences selected from the group consisting of the following amino acid sequences: SEQ ID NO: 1, 3, 5, 7, 9, 11, 13, 15, 17, 19, 21, 23, 25, and 27; preferably, the heavy chain variable has the CDRs identical to that of the amino acid sequence of SEQ ID NO: 1, 3, 5, 7, 9, 11, 13, 15, 17, 19, 21, 23, 25, or 27, and the non-CDRs of the heavy chain variable region have a sequence with at least 80% identity with the non-CDRs of the amino acid sequence: SEQ ID NO: 1, 3, 5, 7, 9, 11, 13, 15, 17, 19, 21, 23, 25, or 27; preferably, the heavy chain variable region has any one amino acid sequence selected from the group consisting of the following amino acid sequences: SEQ ID NO: 1, 3, 5, 7, 9, 11, 13, 15, 17, 19, 21, 23, 25, and 27; and/or
a light chain variable region, the light chain variable region has at least 80% identity with any one amino acid sequence selected from the group consisting of the following amino acid sequences: SEQ ID NO: 2, 4, 6, 8, 10 , 12, 14, 16, 18, 20, 22, 24, 26, 28, 29, 30, 31, 32, 33, 34, 35, and 36; preferably, the light chain variable region has CDRs identical to that of the sequence of SEQ ID NO: 2, 4, 6, 8, 10, 12, 14, 16, 18, 20, 22, 24, 26, 28, 29, 30, 31, 32, 33, 34, 35 or 36, and the non-CDRs of the light chain variable region have a sequence with at least 80% identity with the non-CDRs of SEQ ID NO: 2, 4, 6, 8, 10, 12, 14, 16, 18, 20, 22, 24, 26, 28, 29, 30, 31, 32, 33, 34, 35 or 36; preferably, the light chain variable region has any one amino acid sequence selected from the group consisting of the following amino acid sequences: SEQ ID NO: 2, 4, 6, 8, 10, 12, 14, 16, 18, 20, 22, 24, 26, 28, 29, 30, 31, 32, 33, 34, 35, and 36.

Optionally, the antibody or antigen-binding fragment thereof which comprises a heavy chain variable region and a light chain variable region. The sequence pair of the heavy chain variable region/light chain variable region is selected from the group consisting of the following amino acid sequence pairs: SEQ ID NO:1/2, 3/4, 5/6, 7/8, 9/10, 11/12, 13/14, 15/16, 17/18, 19 /20, 21/22, 23/24, 25/26, 27/28, 21/29, 21/30, 21/31, 21/32, 21/33, 21/34, 21/35, and 21/ 36;
preferably, the sequence pair of the heavy chain variable region/light chain variable region is selected from the group consisting of the following amino acid sequence pairs: SEQ ID NO: 13/14,19/20, 21/22, 23/ 24, 25/26, 27/28, 21/29, 21/30, 21/31, 21/32, 21/33, 21/34, 21/35, and 21/36.

Optionally, the antibody or antigen-binding fragment thereof comprises a heavy chain constant region and/or a light chain constant region, preferably comprising a murine or humanized heavy chain constant region and/or light chain constant region; preferably, the amino acid sequence of the humanized heavy chain constant region is as shown in SEQ ID NO: 117, and the amino acid sequence of the humanized light chain constant region is as shown in SEQ ID NO: 118.

Optionally, the amino acid sequence pair of heavy chain/light chain of the antibody or antigen-binding fragment thereof is selected from the group consisting of the following amino acid sequence pairs: SEQ ID NO:119/120, 121/122 and 123/124.

Optionally, the antibody or antigen-binding fragment thereof, wherein each CDR is defined according to Kabat definition, Chothia definition, Abm definition and/or Contact definition.

Optionally, the antibody or antigen-binding fragment thereof, wherein each CDR is defined according to Kabat definition or Chothia definition. For example, embodiments of the present invention adopted the Kabat definition.

The second aspect of the present invention provides a nucleic acid molecule, the nucleic acid molecule has nucleotide sequences encoding the antibody or antigen-binding fragment thereof as described in the first aspect. The nucleic acid molecules can be synthetic, recombinant, or isolated. Due to the degeneracy of nucleic acid code, multiple nucleic acids will encode the same amino acid and all are covered by it.

The third aspect of the present invention provides an expression vector, the expression vectors comprise the nucleic acid molecules as described in the second aspect.

The fourth aspect of the present invention provides a host cell, the host cell comprise the expression vectors as described in the third aspect.

The fifth aspect of the present invention provides a method of producing antibody or antigen-binding fragment thereof, comprising culturing the host cells as described in the fourth aspect and recovering the antibody or antigen-binding fragment thereof expressed by the method from the culture. The antibody or antigen-binding fragment thereof described herein can be produced from antibody-secreting hybridomas or recombinantly generated cells that have been transformed or transfected with one or more genes encoding antibody or antigen-binding fragment thereof. Cultivate host cell under the condition of expressing nucleic acid to produce antibody, and subsequently recover antibody to produce antibody or antigen-binding portion thereof.

Recombinant expression utilizes the construction of an expression vector containing polynucleotides, which encode antibody or antigen-binding portion thereof. Once the polynucleotides are obtained, vectors for producing antibody can be produced by recombinant DNA techniques well known in the art. The expression vector can include appropriate transcription and translation control signals. This can be achieved by using in vitro recombinant DNA techniques, synthesis techniques, and in vivo gene recombination.

Optionally, the host cell is a prokaryotic cell or a eukaryotic cell. Optionally, the host cell is an E. coli cell, a yeast cell, an insect cell, a plant cell or a mammalian cell.

Optionally, the host cells are Chinese hamster ovary cells (CHO), CHO cell variants, 293 cells or NSO cells. The cell lines include VERO, BHK, HeIa, COS, MDCK, 293F, 293T, 3T3, W138, BT483, Hs578T, HTB2, BT20 and T47D, CRL7030, and HsS78Bst cells. But the cell lines used in the present invention include but are not limited to the above cell lines.

Once the antibody or antigen binding portion of the antibody has been generated through recombinant expression, it can be purified using any method known in the art for the purification of immunoglobulin molecules, Such as, by chromatography, centrifugation, differential solubility, or by any other standard techniques used for protein purification.

The sixth aspect of the present invention provides a pharmaceutical composition comprising at least one antibody or antigen-binding fragment thereof as described in the first aspect, and pharmaceutically acceptable excipients. For example, antibody or antigen binding fragment thereof can be combined with injection water or with saline solution.

The seventh aspect of the present invention provides the use of the antibody or antigen binding fragment thereof as described in the first aspect, or the pharmaceutical composition as described in the sixth aspect, in the preparation of a medicament for the treatment and/or prevention of respiratory syncytial virus infection (RSV) or symptoms related to respiratory syncytial virus (RSV) infection.

The present invention also provides the antibody or antigen-binding fragment thereof as described in the first aspect or the pharmaceutical composition as described in the sixth aspect, for use in the treatment and/or prevention of respiratory syncytial virus (RSV) infection or RSV symptoms related to RSV infection.

The present invention also provides a method for treating and/or preventing respiratory syncytial virus (RSV) infection or symptoms related to RSV infection, comprising administering a therapeutically effective amount of the any one of the antibody or antigen-binding fragment thereof as described in the first aspect or the pharmaceutical compositions described in the sixth aspect to a subject in need thereof.

The present invention also provides use of an active ingredient, the active ingredient is selected from the antibody or antigen-binding fragment thereof described in the first aspect. Wherein the active ingredient is used in the preparation of a detection plate or kit, the detection plate or kit is used to detect respiratory syncytial virus (RSV).

The present invention also provides a detection plate, comprising a substrate and a test strip, wherein the test strip contains the antibody or antigen-binding fragment thereof as described in the first aspect.

The present invention also provides a kit, comprising:
(1) a first container, containing the antibody or antigen-binding fragment thereof as described in the first aspect; and / or
(2) a second container, containing a secondary antibody against the antibody as described in the first aspect;
or, the kit contains the detection plate above.

The RSV antibody provided by the present invention can bind to RSV-F protein with high affinity after humanization modification, and experiments have shown that it exhibits significantly better effect than palivizumab in providing protection against RSV attack.

### Definition

Unless otherwise indicated, any polypeptide chain is herein described as having an amino acid sequence that begins at the N-terminus and terminates at the C-terminus.

The term "antibody and antigen-binding portion thereof" is used herein in its broadest sense, which includes a variety of antibody structures including but not limited to, monoclonal antibodies, polyclonal antibodies, multi-specific antibodies (e.g., bispecific antibodies), and antibody fragments, as long as they exhibit the desired antigen-binding activity.

The term "antibody" as used herein refers to an immunoglobulin molecule consisting of four polypeptide chains, i.e., two heavy (H) and two light (L) chains interconnected by disulfide bonds. Each heavy chain contains a heavy chain variable region (HCVR or VH) and a heavy chain constant region. The heavy chain constant region contains three structural domains, CH1, CH2 and CH3. Each light chain contains a light chain variable region (LCVR or VL) and a light chain constant region. The light chain constant region contains one structural domain (CL1). The VH and VL regions can be further divided into highly variable regions known as complementarity determining region(s) (CDR(s)), which are scattered with more conserved regions known as framework regions (FR). Each VH and VL consists of three CDRs and four FRs arranged in the following order from the amino acid terminus to the carboxyl terminus: FR1, CDR1, FR2, CDR2, FR3, CDR3, FR4, where the three CDRs for VH are HCDR1, HCDR2, and HCDR3, and the three CDRs for VL are LCDR1, LCDR2, and LCDR3. The amino acid assignments for each structural domain are usually consistent with the definition of Kabat Sequences of Proteins of Immunological Interest (National Institutes of Health, Bethesda, Md. (1987 and 1991) or Chothia & Lesk, J. Mol. Biol., 196:901-917 (1987); Chothia et al, Nature, 342:878-883 (1989).

There are five main types of antibodies: IgA, IgD, IgE, IgG, and IgM, and some of these can be further divided into subclasses (isotypes), e.g., IgG1, IgG2, IgG3, IgG4, IgA1, and IgA2.The amino acid sequence at the C-terminal end of the antibody molecule is relatively stable, and this region is called the constant region. The constant region is the same for the same antibody. The constant region of the light chain of an antibody consists of one Ig structural domain; the constant region of the heavy chain consists of 3-4 tandemly linked Ig structural domains and a hinge region for increased flexibility. IgA, IgE, and IgG include three structural domains (CH1, CH2, and CH3), and IgD and IgM include four structural domains (CH1, CH2, CH3, and CH4).

The different types of heavy chain constant structural domains or heavy chain constant regions corresponding to immunoglobulins are called α, δ, ε, γ, and µ. IgG molecules can be degraded by papain into two Fab segments and one Fc segment. The Fab segment consists of the variable region of the light chain, the constant region of the light chain, the variable region of the heavy chain, and the constant region of the heavy chain of the antibody. The variable region is the site of binding to antigen, and therefore the Fab segment is also known as the antigen-binding segment. The Fc segment contains protein sequences common to all antibody molecules and determinants unique to each class. The Fc segment has a variety of biological activities, such as binding to complement, binding to Fc receptors, and passing through the placenta, etc.

As used herein, the term "antigen-binding portion" of an antibody (or simply "antibody portion" or "antibody fragment") refers to one or more fragments of an antibody that retain the ability to bind specifically to an antigen (e.g., RSV-F protein). It has been shown that the antigen-binding function of an antibody can be achieved by certain fragments of a full-length antibody. The term "antigen-binding portion" of an antibody encompasses binding fragments that include (i) Fab fragments, i.e., monovalent fragments consisting of the VL, VH, CL1, and CH1 domains; (ii) F(ab')2 fragments, i.e., bivalent fragments consisting of two F(ab') fragments connected by a disulfide bond in the hinge region; (iii) the Fd fragment consisting of the VH and CH1 structural domains; (iv) the Fv fragment consisting of the VL and VH structural domains of the single arm of the antibody; (v) the dAb fragment consisting of the VH structural domains; and (vi) the CDR. In addition, although, the two structural domains of the Fv fragment, VL and VH, are encoded by different genes, they can be linked together by recombination by a synthetic connector to form separate linked chains in which the VL and VH regions are paired to form a monovalent molecule (referred to as single-chain Fv (scFv)). Such single-chain antibodies are also covered by the term "antigen-binding portion" of the antibody. Other forms of single-chain antibodies, such as bispecific antibodies, are also covered.

Different analyses may be used to determine or roughly estimate CDR regions. Examples of described methods include, but not limited to Kabat definition, Chothia definition, AbM definition, and contact definition. Kabat definition is a standard used for numbering residues in antibodies and commonly used to identify CDR regions. See, e.g., Johnson & Wu, Nucleic Acids Res., 28:214-8 (2000). The Chothia definition is similar to the Kabat definition, except that the Chothia definition takes into account the location of certain structural loop regions. See, e.g., Chothia et al, J. Mol. Biol., 196:901-17 (1986); Chothia et al, Nature, 342:877-83 (1989). AbM definition uses an integrated set of computer programs produced by the Oxford Molecular Group that mimic the structure of antibodies. See, for example, Martin et al, Proc Natl Acad Sci (USA), 86: 9268-9272 (1989); "AbMTM, A Computer Program for Modeling Variable Regions of Antibodies " Oxford, UK; Oxford Molecular, Ltd. AbM definition constructs the modeling of the tertiary structure of an antibody from primary sequences using known databases and the ab initio method, said method are, for example, described in the following literatrue: Samudrala et al, "Ab Initio Protein Structure Prediction Using a Combined Hierarchical Approach " PROTEINS, Structure, Function and Genetics Suppl., 3: 194-198 (1999). The contact definition is based on the analysis of validated complex crystal structures. See, e.g., MacCallum et al, J. Mol. Biol., 5: 732-45 (1996).

The antibodies of the present invention, without limitation as to their source, may be human antibodies, mouse antibodies, rat antibodies, and other antibodies from any animal. It may also be a recombinant antibody such as a chimeric antibody or a humanized antibody. Preferably, the antibody is humanized.

The term "chimeric antibody" means an antibody comprising a variable region of the heavy chain and light chain of a mammal other than a human, such as a mouse antibody, and a constant region of the heavy chain and light chain of a human antibody. Chimeric antibodies can be prepared using known methods. For example, a chimeric antibody can be prepared from the gene of a hybridoma clone, inserted into an appropriate vector, and introduced into a host. Specifically, a cDNA for the variable region (V) region of the antibody is synthesized from the mRNA of the hybridoma using reverse transcriptase. When the DNA encoding the V region of the target antibody is obtained, it is ligated to the DNA encoding the constant region (C region) of the desired human antibody, which is then inserted into an expression vector. Alternatively, the DNA encoding the antibody V region can be inserted into a DNA expression vector comprising the human antibody C region. It is inserted into the expression vector so that it is expressed under the mediation of the expression regulatory region. Next, the chimeric antibody can be expressed by transforming a host cell using the expression vector.

The term "humanized antibody" refers to an antibody that contains at least one, and usually two, nearly complete variable regions, wherein all or nearly all of the corresponding CDR regions are derived from the non-humanized antibody and all or nearly all of the FR regions are derived from the humanized antibody.

The term "affinity" refers to the strength of the sum of the non-covalent interactions between individual binding sites of a molecule (e.g., an antibody) and its binding partner (e.g., an antigen). Unless otherwise specified, as used herein, "binding affinity" refers to the inherent binding affinity of a 1:1 interaction between members of a reaction-binding pair (e.g., antibody and antigen). The affinity of molecule X for its partner Y can typically be expressed by an equilibrium dissociation constant (K_{D}), a dissociation constant (K_{d}), or a binding constant (Kₐ). The affinity can be measured by general methods known in the art, including those described herein. Specific illustrative and exemplary embodiments for measuring binding affinity are described below.

The term "surface plasmon resonance" refers to an optical phenomenon that allows for real-time interactive analysis by detecting a change in protein concentration in a biosensor matrix, for example, using a Biacore^{™} system for analysis.

The term "neutralizing" or "blocking" antibody means an antibody whose binding to the RSV-F protein results in inhibition of the biological activity of the RSV-F protein. Such inhibition of RSV-F protein bioactivity can be assessed by measuring one or more indicators of RSV-F protein bioactivity that are well known in the art, such as RSV-F protein-induced cellular activation and antibody binding to RSV-F protein, etc. (see Examples below).

Sequence similarity of polypeptides also known as sequence identity/homology, is typically measured by sequence analysis software. Protein analysis software utilizes similarity measurements of various substitutions, deletions, and other modifications, including conserved amino acid substitutions, to match similar sequences, e.g., the GCG software includes programs such as Gap and Bestfit, etc, which can be used with default parameters to determine the sequence homology or sequence identity of closely related polypeptides, such as homologous polypeptides from different biological species, see, e.g., GCG 6.1 version. Peptide sequences can also be compared with default or suggested parameters using the FASTA program in GCG 6.1 version. FASTA (e.g., FASTA2 and FASTA3) provides a comparison of the best iterative region between the queried and searched sequences and a sequence identity percentage (Pearson (2000) as above). When comparing the sequences of the present invention with databases containing a large number of sequences originating from different organisms, another preferred computational procedure is the computer program BLAST, in particular BLASTP or TBLASTN, which uses default parameters when comparing.

The term "basic identity" or "substantially identical" when referring to a nucleic acid or fragment thereof, means having nucleotide sequence identity in at least about 80%, preferably in at least about 80%, 85%, 91%, 92%, 93%, 94%, 95%, 96%, 97%, 98%, or 99% of the nucleotide bases when optimally aligned to another nucleic acid (or complementary strand thereof) with appropriate nucleotide substitutions, insertions, or deletions as determined by any of the following sequence identity calculating programs, such as FASTA, BLAST or Gap.

When applied to a peptide, the term "basic identity" or "substantially similar" means that the two peptide sequences have at least 80% sequence identity when optimal alignment with default null weights using a program such as Gap or BESTFIT, etc, more preferably have at least 80%, 85%, 91%, 92%, 93%, 94%, 95%, 96%, 97%, 98% or 99% sequence identity. The difference between the dissimilar residue positions may be an amino acid substitution, deletion or insertion, more preferably the difference between the dissimilar residue positions is a conserved amino acid substitution. A "conservative amino acid substitution" is a substitution in which an amino acid residue is replaced by another amino acid residue containing a side chain (R group) with similar chemical properties (e.g., charge or hydrophobicity). In general, conservative amino acid substitutions do not substantially alter the functional properties of a protein. In cases where two or more amino acid sequences differ due to a conservative substitution, the percent of sequence identity or degree of similarity may be adjusted upwardly to correct for the conservatism of the substitution. Methods for making such adjustments are well known to those skilled in the art. Examples of groups of amino acids containing side chains having similar chemical properties include (1) aliphatic side chains: glycine, alanine, valine, leucine, and isoleucine; (2) aliphatic-hydroxy groups: serine and threonine; (3) amide-containing side chains: asparagine and glutamine; (4) aromatic side chains: phenylalanine, tyrosine, and tryptophan; (5) basic side chains: lysine, arginine, and histidine; (6) acidic side chains: aspartic acid and glutamic acid, and (7) sulfur-containing side chains: cysteine and methionine. Preferred groups of conservative amino acid substitutions are: valine-leucine-isoleucine, phenylalanine-tyrosine, lysine-arginine, alanine-valine, glutamic acid-aspartic acid, and asparagine-glutamine. Alternatively, a conservative substitution may be any variation having a positive value in the PAM250 log-likelihood matrix disclosed in Gonnet et al. (1992) Science256:1443-1445. A "moderately conservative" substitution is any variation having a non-negative value in the PAM250 log-likelihood matrix.

The term "vector" means any molecule or entity (e.g., nucleic acid, plasmid, phage, or virus) used to transfer protein coding information to a host cell.

The term "expression vector" or "expression construct" refers to a vector that is suitable for transformation of a host cell and contains a nucleic acid sequence that directs and/or regulates the expression of one or more heterologous coding regions operably linked thereto. Expression vectors may include, but are not limited to: sequences that affect or regulate transcription, translation; and, if introns are present, sequences that affect RNA splicing of coding regions operably linked thereto.

The terms "host cell", "host cell line" and "host cell culture" can use interchangeably and refer to a cell into which exogenous nucleic acids are introduced, including the progeny of the cell. Host cells include "transformants" and "transformed cells", which include primary transformed cells and progeny derived therefrom (regardless of the number of passages). The filial generation may not have exactly the same nucleic acid content as the parent cell, but may contain mutations. Mutant progeny having the same function or biological activity as that for which they were screened or selected in the original cells are included herein.

The term "transfection" refers to the uptake of foreign or exogenous DNA by a cell, which is "transfected" when the exogenous DNA is introduced into the cell membrane. Various transfection techniques are well known in the art. See, e.g., Graham et al. 1973, Virology 52:456; Sambrook et al. 2001, (Molecular Cloning: A Laboratory Manual); Davis et al. 1986, Basic Methods in Molecular Biology. (Basic Methods in Molecular Biology), Elsevier; Chu et al. 1981, Gene 13: 197. The techniques described may be used to introduce one or more exogenous DNA fractions into a suitable host cell.

The term "treatment" includes therapeutic treatment, prophylactic treatment, and applications in reducing the risk of developing a disease or other risk factors in the treated person. Treatment does not require a complete cure of the disease, but rather includes a program of implementation in which symptoms are alleviated or potential risk factors are mitigated.

The term "prophylaxis" does not require the elimination of 100% of the likelihood of an event. More precisely, it means that the likelihood of an event is reduced in the presence of the compound or method described.

The term "secondary antibody" refers to a second antibody that binds to the antibody, i.e., an antibody to the antibody, and whose main function is to detect the presence of the antibody and amplify the signal of the primary antibody. A secondary antibody is to use antigenic properties of Macromolecular protein of an antibody to immunize a heterologous animal, and is an immunoglobulin produced by the immune system of a heterologous animal that targets the antibody. Secondary antibodies are reactive against all antibodies (e.g., IgG, IgM, or IgA, etc.) of a particular species (e.g., mouse).

### Brief Description of the Figures

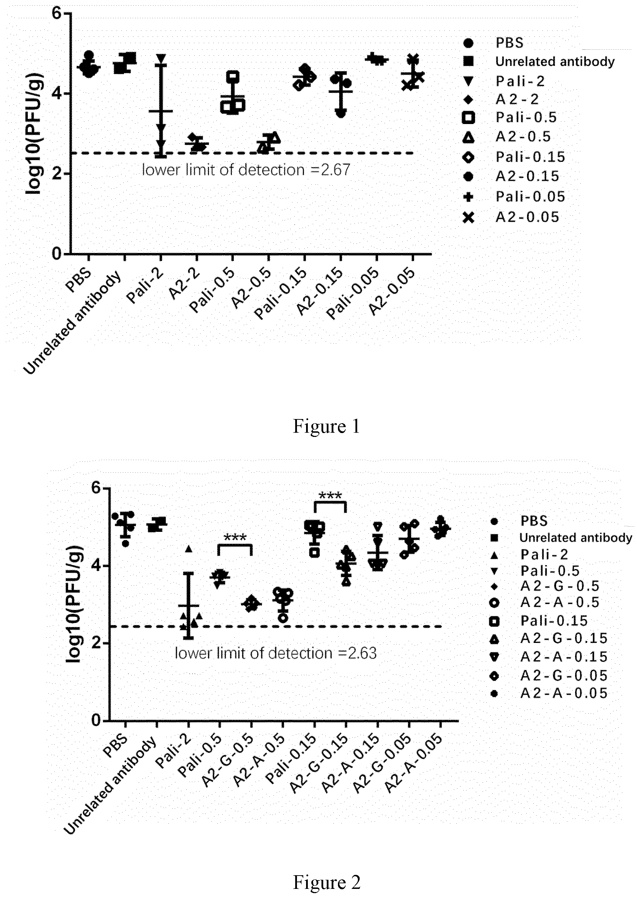

Figure 1 : Results of RSV A2 attack in mice treated with humanized antibodies, the horizontal axis shows the numbering of the groups, in order, PBS, irrelevant antibody, Pali-2, A2-2, Pali-0.5, A2-0.5, Pali-0.15, A2-0.15, Pali-0.05, A2-0.05, wherein PBS is the blank control group, irrelevant antibody is the negative control group, Pali-2, Pali-0.5, Pali-0.15 and Pali-0.05 were positive control Palivizumab groups administered at doses of 2 mg/kg, 0.5 mg/kg, 0.15 mg/kg and 0.05 mg/kg, respectively, andA2-2, A2-0.5, A2-0.15 and A2-0.05 were experimental group A2administered at doses of 2 mg/kg, 0.5 mg/kg, 0.15 mg/kg and 0.05 mg/kg, respectively; the vertical axis is the viral titer (unit: log10(PFU/g)).
Figure 2: Results of RSV A2 attack in mice treated with humanized antibodies with mutations in the CDR region; the horizontal axis shows the numbering of the groups in the following order: PBS, irrelevant antibodies, Pali-2, Pali-0.5, A2-G-0.5, A2-A-0.5, Pali-0.15, A2-G-0.15, A2-A-0.15, A2-G-0.05, A2-G-0.05. A2-A-0.05, wherein PBS was the blank control group, irrelevant antibody was the negative control group, Pali-2, Pali-0.5 and Pali-0.15 were the positive control palivizumab groups administered at the doses of 2 mg/kg , 0.5 mg/kg and 0.15 mg/kg, respectively, and A2-G-0.5, A2-G-0.15 and A2-G- 0.05 were the experimental group A2-G administered at the doses of 0.5 mg/kg, 0.15 mg/kg and 0.05 mg/kg, respectively, and A2-A-0.5, A2-A-0.15 and A2-A-0.05 were the experimental group A2-A administered at the doses of 0.5 mg/kg, 0.15 mg/kg and 0.05 mg/kg, respectively; the vertical axis is the viral titer (in log10(PFU/g)).

### Detailed Description of the Invention

Implementations of the invention will be described in detail below in connection with the examples, but it will be understood by those skilled in the art that the following examples are for the sole purpose of illustrating the invention and should not be regarded as limiting the scope of the invention. Where specific conditions are not indicated in the examples, conventional conditions or conditions recommended by the manufacturer are followed. Where no manufacturer is indicated, the reagents or instruments used are conventional products available commercially.

### Abbreviations

PBS is Phosphate Buffer Solution.

PBST is PBS solution with Tween-20.

BSA is Bovine Serum Albumin. BSA Blocking Solution is formulated from clear albumin.

TMB refers to 3,3',5,5'-Tetramethylbenzidine solution.

MEM medium refers to Minimum Essential Medium, a commonly used medium in animal cell culture.

FBS is Fetal Bovine Serum.

ND (Not Detected) is the antibody does not exhibit *in vitro* cell-neutralizing activity under the experimental conditions.

### Example 1: Screening of Hybridoma Cell Strains

### Production of Recombinant RSV-F Protein

Based on the gene sequence of RSV A2 strain fusion (F) protein in NCBI database, a expressed gene with added His tag was chemically synthesized. This gene was then cloned into the pCDNA3.1(+) expression vector to construct a plasmid for the expression of RSV-F protein. After transfection into 293F cells, the cells were cultured, and the cell culture supernatant containing the expressed protein was collected. The supernatant was purified by nickel column (Cytiva) after concentration, obtaining the RSV-F protein.

### Construction of Hybridoma Cells

40 female BALB/C mice aged 6-8 weeks were immunized after a week of feeding. For the initial immunization, RSV-F protein was used. Before immunization, 1 ml of PBS was used to resuspend Sigma Adjuvant System adjuvant, and then equal volume of 1 ml of RSV-F protein (1mg/ml) as antigen was added to the adjuvant suspension. The mixture was emulsified and injected into the abdomen and subcutaneously at two points, with each mouse receiving 200µl per injection. Booster immunizations were performed on days 21 and 42 after the first immunization, following the same procedure. Blood samples were collected on the 14th day after the second and third immunizations to detect antibody titers. Once the titer reached 1:80000, spleens were harvested for fusion. 72 hours before fusion, an additional booster immunization was administered by intraperitoneal injection of RSV-F antigen protein at a dose of 200µl per mouse.

Spleen cells from immunized mice were fused with SP2/0 myeloma cells (purchased from the American Type Culture Collection). The spleen was ground to obtain a splenic cell suspension, and these cells were mixed in a 1:1 ratio with SP2/0 myeloma cells in logarithmic growth phase. The two cell types were fused using electrofusion to obtain hybridoma cells, and the hybridoma cell solution was diluted to a concentration of 5000-10000 cells/ml and evenly distributed in a 96-well plate. The fusion medium was DMEM complete screening medium containing HAT and 20% FBS.

### Screening of Hybridoma Cells using ELISA and Biacore Methods

(1) To evaluate the immune efficacy (i.e., screening for positive hybridoma parental cell lines) using the ELISA method, the steps are as follows: a. RSV-F recombinant protein was diluted to 1µg/mL with coating buffer (50 mM carbonate, pH 9.6) and added to the Elisa plate (100µL per well), which was then incubated overnight at 4°C; b. Set the plate washer to perform two wash cycles. Wash each well twice with PBST (10 mM PBS + 0.05% Tween 20). After washing, add 200µL of 2% BSA blocking solution to each well to block ELISA plate and incubate at 37°C for 2 hours; c. Set the plate washer for two additional wash cycles. Wash each well twice with PBST (10 mM PBS + 0.05% Tween 20). After washing, add 100µL of mouse serum or hybridoma supernatant, diluted with 2% BSA blocking solution, to each well. Include two replicate wells and add a negative control. Incubate at 37°C for 1 hour; d. After washing 3 times with PBST, a secondary antibody (Goat anti-Mouse IgG (H+L) Secondary Antibody, HRP) (Invitrogen) was diluted 10000 times with 2% BSA blocking buffer, 100µL of the solution was added to each well, and the plate was incubated for 1 hour at 37°C; e. After washing 4 times with PBST, add 100µL of single-component TMB chromogenic solution (Solarbio) to each well. Incubate in the dark for 5-15 minutes; f. Terminate the reaction: Terminate the reaction by adding 50µL of 2M H₂SO₄ to each well; g. Reading: Put the ELISA plate into a microplate reader for reading, and the detection wavelength is OD450nm.
(2) To determine the binding activity of positive hybridoma cell lines using Biacore, the steps are as follows: a. Centrifuge the supernatant of positive hybridoma parent cells at 12000 rpm for 10 minutes, for further use; b. Use the Biacore 8K control software and a CM5 chip for the experiment. c. Write the detection program: Set up three cycles, then specify the capture time (capturing antibodies from the supernatant) as 300s at a flow rate of 30µL/min; Analyze the sample concentration at 40nM with a binding time of 120s at 30µL/min. The dissociation time was set at 120s, and the regeneration was done with 10 mM glycine-HCl at 30 µL/min for 30s. Fill in the sample numbers and corresponding protein concentrations in the list. Place the appropriate reagents in the designated positions as per the program, cover the membrane, and insert the 96-well plate into the sample chamber. Start the program; d. Analyze the results and identify hybridoma cell lines with an affinity greater than nM (10⁻⁹) level.

### Sequencing of Hybridoma Subclone Cell Strains

Positive hybridoma cells obtained through the above methods were cultured, and once the cells exhibited good growth, a limited dilution method was used for cloning.

Sequencing of the cloned cells involved the following steps: a. Cells were collected, treated with Trizol (Tiangen Biotech Co., Ltd), and total RNA was extracted ; b. Reverse transcription: 8µL RNA (50ng-5pg) was mixed with 1µL Oligo(dT)18 primer in a 0.2 mL PCR tube, incubated at 65°C for 5 minutes, and then incubateded on ice for 2 minutes. Next, 10µL 2×ES Reaction Mix and 1µL RT Enzyme Mix were added, and the mixture was incubated at 42°C for 30 minutes followed by a 5-second incubation at 85°C to terminate transcription; c. PCR amplification was performed under the following conditions: 94°C for 5 minutes; 30 cycles of 94°C for 30s, 55°C for 30s, and 72°C for 45s; 72°C for 10 minutes; and 4°C for 5 minutes; d. The amplified products were subjected to sequencing.

Results: Sequencing results of subcloned hybridoma cells are presented in Table 2.

**Table 2: Sequencing Results of Subcloned Hybridoma Cells**

| Antibody | | Sequence of the heavy chain variable region | | | Sequence of the light chain variable region | | |
|---|---|---|---|---|---|---|---|
| R3-16A10 | DNA sequence | SEQ ID NO:91 | | | SEQ ID NO:92 | | |
| | Amino acid sequence | SEQ ID NO:1 | | | SEQ ID NO:2 | | |
| | CDR region | SEQ ID NO:37 | SEQ ID NO:38 | SEQ ID NO:39 | SEQ ID NO:40 | SEQ ID NO:41 | SEQ ID NO:42 |
| R10-10B2 | DNA | SEQ ID NO:93 | | | SEQ ID NO:94 | | |
| | sequence | | | | | | |
| | Amino acid sequence | SEQ ID NO:3 | | | SEQ ID NON | | |
| | CDR region | SEQ ID NO:43 | SEQ ID NO:44 | SEQ ID NO:45 | SEQ ID NO:46 | SEQ ID NO:47 | SEQ ID NO:48 |
| R18-11B1 | DNA sequence | SEQ ID NO:95 | | | SEQ ID NO:96 | | |
| | Amino acid sequence | SEQ ID NO: 5 | | | SEQ ID NO: 6 | | |
| | CDR region | SEQ ID NO:49 | SEQ ID NO:50 | SEQ ID NO: 51 | SEQ ID NO:52 | SEQ ID NO:53 | SEQ ID NO:54 |
| R1-11C3 | DNA sequence | SEQ ID NO:97 | | | SEQ ID NO:98 | | |
| | Amino acid sequence | SEQ ID NO: 7 | | | SEQ ID NO: 8 | | |
| | CDR region | SEQ ID NO:55 | SEQ ID NO:56 | SEQ ID NO:57 | SEQ ID NO:58 | SEQ ID NO:59 | SEQ ID NO:60 |
| R10-6A11 | DNA sequence | SEQ ID NO:99 | | | SEQ ID NO:100 | | |
| | Amino acid sequence | SEQ ID NO:9 | | | SEQ ID NO:10 | | |
| | CDR region | SEQ ID NO:61 | SEQ ID NO:62 | SEQ ID NO:63 | SEQ ID NO:64 | SEQ ID NO:65 | SEQ ID NO:66 |
| R18-22D7 | DNA sequence | SEQ ID NO:101 | | | SEQ ID NO:102 | | |
| | Amino acid sequence | SEQ ID NO:11 | | | SEQ ID NO:12 | | |
| | CDR region | SEQ ID NO:67 | SEQ ID NO:68 | SEQ ID NO:69 | SEQ ID NO:70 | SEQ ID NO:71 | SEQ ID NO:72 |
| R10-19B2 | DNA sequence | SEQ ID NO:103 | | | SEQ ID NO:104 | | |
| | Amino acid sequence | SEQ ID NO:13 | | | SEQ ID NO:14 | | |
| | CDR region | SEQ ID NO:73 | SEQ ID NO:74 | SEQ ID NO:75 | SEQ ID NO:76 | SEQ ID NO:77 | SEQ ID NO:78 |
| R3-39A3 | DNA sequence | SEQ ID NO:105 | | | SEQ ID NO:106 | | |
| | Amino acid sequence | SEQ ID NO:15 | | | SEQ ID NO:16 | | |
| | CDR region | SEQ ID NO:79 | SEQ ID NO:80 | SEQ ID NO: 81 | SEQ ID NO:82 | SEQ ID NO:83 | SEQ ID NO: 84 |
| R3-38B6 | DNA sequence | SEQ ID NO:107 | | | SEQ ID NO:108 | | |
| | Amino acid sequence | SEQ ID NO:17 | | | SEQ ID NO:18 | | |
| | CDR region | SEQ ID NO:85 | SEQ ID NO:86 | SEQ ID NO:87 | SEQ ID NO:88 | SEQ ID NO:89 | SEQ ID NO:90 |

### Example 2: Preparation of Chimeric Antibodies

### Construction and Expression of Chimeric Antibodies

Chimeric antibodies were constructed using the fusion PCR method. The coding genes for the variable region of the mouse-derived antibody from Example 1 were linked with that of the constant region of the human-derived antibody (heavy chain constant region amino acid sequence: SEQ ID NO: 117, light chain constant region amino acid sequence: SEQ ID NO: 118) to construct the fusion gene, then the fusion gene was amplified, cloned into the pCDNA3.1(+) expression vector to construct the expression plasmid of chimeric antibody, and transfected into 293F cells. After culturing, the supernatant containing the expressed antibodies was collected, concentrated, and purified using Protein A (Cytiva) to obtain the corresponding chimeric antibodies.

### Affinity Determination of Chimeric Antibodies

Antibodies were captured at a flow rate of 10µl/min for 60s. Antigen binding occurred at a flow rate of 30µl/min for 120s, followed by dissociation at 30µl/min for 1800s. Regeneration was performed at a flow rate of 30µl/min for 30s. After the experiment ended, the results were processed using analysis software. The results are shown in Table 2, indicating that the chimeric antibodies exhibited high-affinity binding to the RSV-F protein. Experimental data demonstrated that the humanized antibodies exhibited affinity to RSV-F protein higher than nM (10⁻⁹).

**Table 2: Affinity Results of Chimeric Antibodies Binding to RSV-F Protein**

| Antibody | Affinity constant Ka (1/Ms) | Dissociation constant Kd (1/s) | Equilibrium dissociation constant K_{D} (M) |
|---|---|---|---|
| R10-6A11QH | 1.03E+06 | 3.39E-04 | 3.29E-10 |
| R10-19B2QH | 2.23 E+06 | 8.58E-05 | 3.85E-11 |
| R3-16A10QH | 5.37E+05 | 2.69E-05 | 5.00E-11 |
| R10-10B2QH | 7.53E+05 | 3.68E-05 | 4.89E-11 |
| R18-11B1QH | 8.24E+05 | 1.69E-04 | 2.05E-10 |
| R1-11C3QH | 5.49E+05 | 1.41E-05 | 2.56E-11 |
| R3-38B6QH | 4.95E+05 | 3.02E-05 | 6.10E-11 |
| R18-22D7QH | 3.57E+05 | 1.27E-03 | 3.54E-9 |
| R3-39A3QH | 6.73E+05 | 1.15E-04 | 1.71E-10 |

### In Vitro Cell Activity Determination of Chimeric Antibodies

Using a volume of 50µL per well, the antibody was added in a threefold serial dilution to the culture medium of HEp-2 cells (ATCC CCL-23) in a 96-well microtiter plate. Subsequently, 50µL of samples containing RSV viruses (including RSV A2, RSV A Long, RSV B9320) at certain dilutions were added to each well. A positive control with virus but without antibody and a negative control with antibody but without virus were also included. The plate was incubated in a 37°C, 5% CO₂ incubator for 2 hours. Hep-2 cell suspension (2×10⁵ cells/mL) was added to each well with 100µL, and the plates were further incubated and cultured for 5 days at 37°C in a 5% CO₂ incubator. The neutralizing activity of antibodies was determined by indirect ELISA.

The formula for calculating the neutralization activity (%) at different concentrations of neutralizing antibodies is as follows: Neutralization Activity (%) = 100 - (Test well reading - Cell control average) / (Virus control average - Cell control average) × 100

Then the non-linear curve fitting analysis was performed using GraphPad Prism (version 5) to obtain the IC₅₀ values for pure neutralizing antibodies.

The *in vitro* cell activity results of chimeric antibodies are shown in Table 3. The data demonstrates that the chimeric antibodies discovered in this invention have significantly lower IC₅₀ values in neutralizing RSV A2, RSV A Long, and RSV B9320 compared to palivizumab.

**Table 3: IC₅₀ Results of Chimeric Antibodies in Neutralizing RSV A2, RSV A Long, and RSV B9320**

| Antibody | RSV A2 Absolute IC₅₀ (ng/mL) | RSV A Long Absolute IC₅₀ (ng/mL) | RSV B9320 Absolute IC₅₀ (ng/mL) |
|---|---|---|---|
| Palivizumab | 1056 | 231.8 | 1067 |
| R10-6A11QH | 7.446 | 19.85 | 265.5 |
| R10-19B2QH | 11.96 | 10.21 | 21.03 |
| R18-22D7QH | 29.99 | 68.25 | 8.932 |
| R3-16A10QH | 11.76 | 25.75 | 72.61 |
| R10-10B2QH | 96.81 | 214.2 | 91.49 |
| R18-11B1QH | 100.8 | 43.23 | ND |
| R1-11C3QH | 117.9 | 315.8 | 66.63 |
| R3-38B6QH | 17.62 | 73.31 | ND |
| R3-39A3QH | 5.919 | ND | ND |

### Example 3: Humanization of Antibody R10-19B2QH

### Preparation of Humanized Antibodies

Retaining the CDR of mouse, the framework regions (Fr) of R10-19B2QH were subjected to humanization through grafting. After humanization, the variable regions of the heavy chain and light chain are as shown in Table 4:

**Table 4: Variable Regions of the Heavy and Light Chains after Humanization of Chimeric Antibody**

| Antibody | The sequence of the heavy chain variable region | The sequence of the light chain variable region |
|---|---|---|
| A1 | SEQ ID NO:19 | SEQ ID NO:20 |
| A2 | SEQ ID NO:21 | SEQ ID NO:22 |
| A3 | SEQ ID NO:23 | SEQ ID NO:24 |
| A4 | SEQ ID NO:25 | SEQ ID NO26 |
| A5 | SEQ ID NO:27 | SEQ ID NO:28 |

### Cell Activity Experiment of Humanized Antibodies

The cell neutralization activity of the humanized antibodies mentioned in Table 4 was assessed (see the methods described in Example 2). The results are presented in Table 5, indicating that the humanized antibodies developed in this invention, based on R10-19B2QH, exhibit significantly lower IC₅₀ values in neutralizing RSV A2, RSV A Long, and RSV B9320 compared to palivizumab.

**Table 5: IC₅₀ Results of Humanized Antibodies Based on R10-19B2QH in Neutralizing RSV A2, RSV A Long, and RSV B9320**

| Antibody | RSV A2 Absolute IC₅₀ (ng/mL) | RSV A Long Absolute IC₅₀ (ng/mL) | RSV B9320 Absolute IC₅₀ (ng/mL) |
|---|---|---|---|
| Palivizumab | 3043 | 1587 | 481.1 |
| A1 | 326.9 | 179.3 | ND |
| A2 | 220.1 | 97.02 | 181.2 |
| A3 | 268.7 | 154.5 | ND |
| A4 | 241.4 | 125.1 | ND |
| A5 | 177.1 | 30.61 | 170.8 |

### Animal Efficacy Experiment of Humanized Antibodies

Mice were grouped and injected with antibodies. Prepare 7-9-week-old mice, randomly divide them into groups of 3 each, feed for 1 week, and then categorize them into the following groups: positive control palivizumab group (doses of 2 mg/kg, 0.5 mg/kg, 0.15 mg/kg, 0.05 mg/kg); experimental group A2 (doses of 2 mg/kg, 0.5 mg/kg, 0.15 mg/kg, 0.05 mg/kg); blank control PBS group; and negative control unrelated antibody group (dose of 2 mg/kg). One day before virus infection (-1d), intraperitoneal injection of 100µL of the antibody was administered.

Intranasal Administration: On the second day (0d) after anesthetizing the mice with isoflurane, each mouse received intranasal administration of 100µL of RSV A2 virus (10⁷ PFU/mL).

Lung Collection for determination: Five days after infection (5d), mouse lung tissues were placed in 1 mL of MEM culture medium (Gibco). The lung tissues were homogenized using a cell grinder and mesh sieve. After centrifugation at 2000rpm for 2.5 hours at 4°C, the supernatant was collected. The virus titer in the supernatant of the lung homogenate was detected. The first well was diluted 2-fold, and 100µL was added to a 96-well plate for further 1:2 gradient dilution of the test samples. Subsequently, 100µL of Hep2 cells at a concentration of 2.5 × 10⁵ cells/mL was added, and the plate was incubated in a CO₂ incubator for 5-6 days. After observing the cell changes, RSV lesion-positive wells were detected by ELISA.

Virus titers [log10(PFU/g)] in the lung tissue homogenate supernatant were calculated based on the number of positive wells.

Results: see Figure 1. The data in figure 1 indicates that A2 performs better than palivizumab in providing protection against RSV attacks.

Example 4: CDR Modification of Humanized Antibody A2 CDR Modification of Humanized Antibody A2

The amino acid sequence of the light chain of humanized antibody A2 is as follows:

DIVMTQSPLSLPVTPGEPASISCRSSKSLLHSNGITYLCWFLQK
PGQSPQLLIYQMSNLASGVPDRFSGSGSGTDFTLKISRVEAEDVGV
YYCAQNLELWTFGQGTRLEIKRTVAAPSVFIFPPSDEQLKSGTASV
VCLLNNFYPREAKVQWKVDNALQSGNSQESVTEQDSKDSTYSLS
STLTLSKADYEKHKVYACEVTHQGLSSPVTKSFNRGEC (SEQ ID
NO:119)

The amino acid sequence of the heavy chain of humanized antibody A2 is as follows:

QVTLKESGPALVKPTQTLTLTCTFSGFSLSTSGMGVSWIRQPP
GKALEWLAHIYWDDDKNFNPSLRSRLTITKDPSKNQVVLTMTNM
DPVDTATYFCARKLYNDYDYILDYWGQGTTVTVSSASTKGPSVFP
LAPSSKSTSGGTAALGCLVKDYFPEPVTVSWNSGALTSGVHTFPAV
LQSSGLYSLSSVVTVPSSSLGTQTYICNVNHKPSNTKVDKRVEPKS
CDKTHTCPPCPAPELLGGPSVFLFPPKPKDTLYITREPEVTCVVVD
VSHEDPEVKFNWYVDGVEVHNAKTKPREEQYNSTYRVVSVLTVL
HQDWLNGKEYKCKVSNKALPAPIEKTISKAKGQPREPQVYTLPPS
REEMTKNQVSLTCLVKGFYPSDIAVEWESNGQPENNYKTTPPVLD
SDGSFFLYSKLTVDKSRWQQGNVFSCSVMHEALHNHYTQKSLSLS
PGK (SEQ ID NO:120)

Site-Specific Saturation Mutagenesis of Cysteine Residue in Light Chain CDR1 Region: Using the fusion PCR method, site-specific saturation mutagenesis was performed on the cysteine residue at position 61 of the light chain plasmid of humanized antibody A2. The specific mutated position is indicated by an underline below:
The amino acid sequence of the light chain variable region of humanized antibody A2 is as follows:

MDMRVPAQLLGLLLLWLSGARCDIVMTQSPLSLPVTPGEPASI
SCRSSKSLLHSNGITYLCWFLQKPGQSPQLLIYQMSNLASGVPDRF
SGSGSGTDFTLKISRVEAEDVGVYYCAQNLELWTFGQGTRLEIK
(SEQ ID NO:22)

Construction of expression plasmids for mutant antibodies. After transfection into 293F cells, the cells were cultured, and the supernatant containing the expressed antibodies was collected. After concentration, the antibodies were purified using Protein A (Cytiva). Eight mutant antibodies with cysteine mutations in the CDR1 of the light chain were obtained. The sequences of each antibody after CDR modification based on humanized antibody A2 are shown in Table 6:

**Table 6: Sequences of Antibodies After CDR Modification Based on Humanized Antibody A2**

| Antibody | The sequence of the light chain variable region | LCDR1 |
|---|---|---|
| A2-G | SEQ ID NO:29 | SEQ ID NO:109 |
| A2-I | SEQ ID NO:30 | SEQ ID NO:110 |
| A2-A | SEQ ID NO:31 | SEQ ID NO:111 |
| A2-M | SEQ ID NO:32 | SEQ ID NO:112 |
| A2-Q | SEQ ID NO:33 | SEQ ID NO:113 |
| A2-N | SEQ ID NO:34 | SEQ ID NO:114 |
| A2-V | SEQ ID NO:35 | SEQ ID NO:115 |
| A2-P | SEQ ID NO:36 | SEQ ID NO:116 |

The amino acid sequence of the light chain of A2-G obtained after the mutation of humanized antibody A2:

DIVMTQSPLSLPVTPGEPASISCRSSKSLLHSNGITYLGWFLQK
PGQSPQLLIYQMSNLASGVPDRFSGSGSGTDFTLKISRVEAEDVGV

YYCAQNLELWTFGQGTRLEIKRTVAAPSVFIFPPSDEQLKSGTASV
VCLLNNFYPREAKVQWKVDNALQSGNSQESVTEQDSKDSTYSLS
STLTLSKADYEKHKVYACEVTHQGLSSPVTKSFNRGEC (SEQ ID
NO:121)

The amino acid sequence of the heavy chain of A2-G obtained after mutation of humanized antibody A2:

QVTLKESGPALVKPTQTLTLTCTFSGFSLSTSGMGVSWIRQPP
GKALEWLAHIYWDDDKNFNPSLRSRLTITKDPSKNQVVLTMTNM
DPVDTATYFCARKLYNDYDYILDYWGQGTTVTVSSASTKGPSVFP
LAPSSKSTSGGTAALGCLVKDYFPEPVTVSWNSGALTSGVHTFPAV
LQSSGLYSLSSVVTVPSSSLGTQTYICNVNHKPSNTKVDKRVEPKS
CDKTHTCPPCPAPELLGGPSVFLFPPKPKDTLYITREPEVTCVVVD
VSHEDPEVKFNWYVDGVEVHNAKTKPREEQYNSTYRVVSVLTVL
HQDWLNGKEYKCKVSNKALPAPIEKTISKAKGQPREPQVYTLPPS
REEMTKNQVSLTCLVKGFYPSDIAVEWESNGQPENNYKTTPPVLD
SDGSFFLYSKLTVDKSRWQQGNVFSCSVMHEALHNHYTQKSLSLS
PGK (SEQ ID NO:122)

The amino acid sequence of the light chain of A2-A obtained after mutation of humanized antibody A2:

DIVMTQSPLSLPVTPGEPASISCRSSKSLLHSNGITYLAWFLQK
PGQSPQLLIYQMSNLASGVPDRFSGSGSGTDFTLKISRVEAEDVGV
YYCAQNLELWTFGQGTRLEIKRTVAAPSVFIFPPSDEQLKSGTASV
VCLLNNFYPREAKVQWKVDNALQSGNSQESVTEQDSKDSTYSLS
STLTLSKADYEKHKVYACEVTHQGLSSPVTKSFNRGEC (SEQ ID
NO:123)

The amino acid sequence of the heavy chain of A2-A obtained after mutation of humanized antibody A2:

QVTLKESGPALVKPTQTLTLTCTFSGFSLSTSGMGVSWIRQPP

GKALEWLAHIYWDDDKNFNPSLRSRLTITKDPSKNQVVLTMTNM
DPVDTATYFCARKLYNDYDYILDYWGQGTTVTVSSASTKGPSVFP
LAPSSKSTSGGTAALGCLVKDYFPEPVTVSWNSGALTSGVHTFPAV
LQSSGLYSLSSVVTVPSSSLGTQTYICNVNHKPSNTKVDKRVEPKS
CDKTHTCPPCPAPELLGGPSVFLFPPKPKDTLYITREPEVTCVVVD
VSHEDPEVKFNWYVDGVEVHNAKTKPREEQYNSTYRVVSVLTVL
HQDWLNGKEYKCKVSNKALPAPIEKTISKAKGQPREPQVYTLPPS
REEMTKNQVSLTCLVKGFYPSDIAVEWESNGQPENNYKTTPPVLD
SDGSFFLYSKLTVDKSRWQQGNVFSCSVMHEALHNHYTQKSLSLS
PGK (SEQ ID NO:124)

### Affinity Determination of Humanized Antibody A2 Variants

The affinity of humanized mutant antibodies was assessed (see the methods described in Example 2). The results are presented in Table 7, indicating that humanized mutant antibodies exhibit high-affinity binding to the RSV-F protein.

**Table 7: Affinity Results of Humanized Mutant Antibodies Binding to RSV-F Protein**

| Antibody | K_{D}(nM) | Ka(M-1s-1) | Kd(s-1) |
|---|---|---|---|
| Palivizumab | 6.28E-11 | 1.20e+6 | 7.55e-5 |
| A2-G | 7E-11 | 1.97e+6 | 1.38e-4 |
| A2-I | 5.28E-11 | 3.07e+6 | 1.62e-4 |
| A2-A | 5.09E-11 | 2.30e+6 | 1.17e-4 |
| A2-M | 5.97E-11 | 2.3 1 e+6 | 1.38e-4 |
| A2-Q | 6.98E-11 | 2.38e+6 | 1.66e-4 |
| A2-N | 1.02E-10 | 1.61e+6 | 1.65e-4 |
| A2-V | 5.8E-11 | 2.78e+6 | 1.61e-4 |
| A2-P | 4.38E-11 | 3.57e+6 | 1.56e-4 |

### Cell Activity Experiment of Humanized Antibody A2 Variants

The cell neutralization activity of humanized mutant antibodies was assessed (see the methods described in Example 2). The results are presented in Table 8, indicating that humanized mutant antibodies discovered in this invention exhibit significantly lower IC₅₀ values in neutralizing RSV A2, RSV A Long, and RSV B9320 compared to palivizumab.

**Table 8: IC₅₀ Results of Humanized Mutant Antibodies in Neutralizing RSV A2, RSV A Long, and RSV B9320**

| Antibody | RSV A2 Absolute IC₅₀ | RSV AL Absolute IC₅₀ | RSV B9320 Absolute IC₅₀ |
|---|---|---|---|
| Palivizumab | 847.7 | 760.4 | 281.6 |
| A2-G | 73.68 | 38.51 | 152.5 |
| A2-I | 109.5 | 82.32 | ND |
| A2-A | 79.82 | 50.88 | 162.2 |
| A2-M | 175.6 | 111.2 | 462.2 |
| A2-Q | 110.2 | 51.7 | 247.8 |
| A2-N | 141.1 | 65.07 | 739.6 |
| A2-V | 90.84 | 84.15 | 166.6 |
| A2-P | 174.5 | 161.8 | 171.4 |

### Animal Efficacy Experiment of Humanized Antibody A2 Variants

Mice were grouped and injected with antibodies. Prepare 7-9-week-old mice, randomly divide them into groups of 5 each, feed for 1 week, and then categorize them into the following groups: positive control palivizumab group (doses of 2mg/kg, 0.5 mg/kg, 0.15 mg/kg); experimental group A2-A (doses of 0.5 mg/kg, 0.15 mg/kg, 0.05 mg/kg); experimental group A2-G (doses of 0.5 mg/kg, 0.15 mg/kg, 0.05 mg/kg); blank control PBS group; and negative control unrelated antibody group (dose of 2 mg/kg). One day before virus infection (-1d), intraperitoneal injection of 100µL of the antibody was administered.

Intranasal Administration: On the second day (0d) after anesthetizing the mice with isoflurane, each mouse received intranasal administration of 100 µL of RSV A2 virus (10⁷ PFU/mL).

Lung Collection for determination: Five days after infection (5d), mouse lung tissues were placed in 1mL of MEM culture medium (Gibco). The lung tissues were homogenized using a cell grinder and mesh sieve. After centrifugation at 2000 rpm for 2.5 hours at 4°C, the supernatant was collected. The virus titer in the supernatant of the lung homogenate was detected. The first well was diluted 2-fold, and 100 µL was added to a 96-well plate for further 1:2 gradient dilution of the test samples. Subsequently, 100 µL of Hep2 cells at a concentration of 2.5 × 10⁵ cells/mL was added, and the plate was incubated in a CO₂ incubator for 5-6 days. After observing the cell changes, RSV lesion-positive wells were detected by ELISA.

Virus titers [log10(PFU/g)] in the lung tissue homogenate supernatant were calculated based on the number of positive wells.

Results: see Figure 2. The data in figure 2 shows that A2-G and A2-A perform better than palivizumab in providing protection against RSV attacks.

The present invention has been described through the above examples, but it should be understood that the above examples are only for illustrative and explanatory purposes and do not intend to limit the scope of the invention described therein. Furthermore, those skilled in the art can understand that the present invention is not limited to the above examples, and further modifications and variations can be made based on the teachings of the present invention, all of which fall within the scope of protection required by the claims and their equivalents.

## Claims

1. An antibody or antigen-binding fragment thereof, which specifically binds to respiratory syncytial virus fusion protein (RSV-F) and/or neutralizes respiratory syncytial virus, as determined by surface plasmon resonance, the antibody or antigen-binding fragment thereof specifically binds to RSV-F with an equilibrium dissociation constant (K_{D}) no higher than 10⁻⁹ M.

2. An antibody or antigen-binding fragment thereof, which specifically binds to respiratory syncytial virus fusion protein (RSV-F) and/or neutralizes respiratory syncytial virus, the antibody or antigen-binding fragment thereof comprises:
three heavy chain complementarity determining regions (CDRs) (HCDR1, HCDR2 and HCDR3) contained within the amino acid sequence SEQ ID NO: 1, 3, 5, 7, 9, 11, 13, 15 or 17; and/or
three light chain complementarity determining regions(CDRs) (LCDR1, LCDR2 and LCDR3) contained within the amino acid sequence SEQ ID NO: 2, 4, 6, 8, 10, 12, 14, 16, 18, 29, 30, 31, 32, 33, 34, 35 or 36.

3. An antibody or antigen-binding fragment thereof, which specifically binds to respiratory syncytial virus fusion protein (RSV-F) and/or neutralizes respiratory syncytial virus, the antibody or antigen-binding fragment thereof comprises:
a) a HCDR3 functional region, the HCDR3 functional region has any one amino acid sequence selected from the group consisting of the following amino acid sequences: SEQ ID NO: 39, 45, 51, 57, 63, 69, 75, 81, and 87; and
b) a LCDR3 functional region, the HCDR3 functional region has any one amino acid sequence selected from the group consisting of the following amino acid sequences: SEQ ID NO: 42, 48, 54, 60, 66, 72, 78, 84, and 90.

4. The antibody or antigen-binding fragment thereof according to claim 3, further comprising:
c) a HCDR1 functional region, the HCDR1 functional region has any one amino acid sequence selected from the group consisting of the following amino acid sequences: SEQ ID NO: 37, 43, 49, 55, 61, 67, 73, 79, and 85;
d) a LCDR1 functional region, the LCDR1 functional region has any one amino acid sequence selected from the group consisting of the following amino acid sequences: SEQ ID NO: 40, 46, 52, 58, 64, 70, 73, 79, 85, 109, 110, 111, 112, 113, 114, 115 and 116;
e) a HCDR2 functional region, the HCDR2 functional region has any one amino acid sequence selected from the group consisting of the following amino acid sequences: SEQ ID NO: 38, 44, 50, 56, 62, 68, 74, 80, and 86; and
f) a LCDR2 functional region, the LCDR2 functional region has any one amino acid sequence selected from the group consisting of the following amino acid sequences: SEQ ID NO: 41, 47, 53, 59, 65, 71, 77, 83 and 89.

5. The antibody or antigen-binding fragment thereof according to any one of claims 2-4, comprising:
1) three heavy chain complementarity determining regions (CDRs) (HCDR1, HCDR2 and HCDR3) contained within the amino acid sequence SEQ ID NO:13, and
three light chain complementarity determining regions (CDRs) (LCDR1, LCDR2 and LCDR3) contained within the amino acid sequence SEQ ID NO: 14, 29, 30, 31, 32, 33, 34, 35 or 36; or
2) three heavy chain complementarity determining regions (CDRs) (HCDR1, HCDR2 and HCDR3) contained within the amino acid sequence SEQ ID NO:1, and
three light chain complementarity determining regions (CDRs) (LCDR1, LCDR2 and LCDR3) contained within the amino acid sequence SEQ ID NO:2; or
3) three heavy chain complementarity determining regions (CDRs) (HCDR1, HCDR2 and HCDR3) contained within the amino acid sequence SEQ ID NO:3, and
three light chain complementarity determining regions (CDRs) (LCDR1, LCDR2 and LCDR3) contained within the amino acid sequence SEQ ID NO:4; or
4) three heavy chain complementarity determining regions (CDRs) (HCDR1, HCDR2 and HCDR3) contained within the amino acid sequence SEQ ID NO:5, and
three light chain complementarity determining regions (CDRs) (LCDR1, LCDR2 and LCDR3) contained within the amino acid sequence SEQ ID NO:6; or
5) three heavy chain complementarity determining regions (CDRs) (HCDR1, HCDR2 and HCDR3) contained within the amino acid sequence SEQ ID NO:7, and
three light chain complementarity determining regions (CDRs) (LCDR1, LCDR2 and LCDR3) contained within the amino acid sequence SEQ ID NO:8; or
6) three heavy chain complementarity determining regions (CDRs) (HCDR1, HCDR2 and HCDR3) contained within the amino acid sequence SEQ ID NO:9, and
three light chain complementarity determining regions (CDRs) (LCDR1, LCDR2 and LCDR3) contained within the amino acid sequence SEQ ID NO: 10; or
7) three heavy chain complementarity determining regions (CDRs) (HCDR1, HCDR2 and HCDR3) contained within the amino acid sequence SEQ ID NO: 11, and
three light chain complementarity determining regions (CDRs) (LCDR1, LCDR2 and LCDR3) contained within the amino acid sequence SEQ ID NO: 12; or
8) three heavy chain complementarity determining regions (CDRs) (HCDR1, HCDR2 and HCDR3) contained within the amino acid sequence SEQ ID NO: 15, and
three light chain complementarity determining regions (CDRs) (LCDR1, LCDR2 and LCDR3) contained within the amino acid sequence SEQ ID NO: 16; or
9) three heavy chain complementarity determining regions (CDRs) (HCDR1, HCDR2 and HCDR3) contained within the amino acid sequence SEQ ID NO: 17, and
three light chain complementarity determining regions (CDRs) (LCDR1, LCDR2 and LCDR3) contained within the amino acid sequence SEQ ID NO: 18.

6. The antibody or antigen-binding fragment thereof according to claim 5, comprising:
1) comprising a HCDR1 shown in SEQ ID NO:37,
comprising a HCDR2 shown in SEQ ID NO:38,
comprising a HCDR3 shown in SEQ ID NO:39,
comprising a LCDR1 shown in SEQ ID NO:40,
comprising a LCDR2 shown in SEQ ID NO:41, and
comprising a LCDR3 shown in SEQ ID NO:42; or
2) comprising a HCDR1 shown in SEQ ID NO:43,
comprising a HCDR2 shown in SEQ ID NO:44,
comprising a HCDR3 shown in SEQ ID NO:45,
comprising a LCDR1 shown in SEQ ID NO:46,
comprising a LCDR2 shown in SEQ ID NO:47, and
comprising a LCDR3 shown in SEQ ID NO:48; or
3) comprising a HCDR1 shown in SEQ ID NO:49,
comprising a HCDR2 shown in SEQ ID NO:50,
comprising a HCDR3 shown in SEQ ID NO:51,
comprising a LCDR1 shown in SEQ ID NO:52,
comprising a LCDR2 shown in SEQ ID NO:53, and
comprising a LCDR3 shown in SEQ ID NO:54; or
4) comprising a HCDR1 shown in SEQ ID NO:55,
comprising a HCDR2 shown in SEQ ID NO:56,
comprising a HCDR3 shown in SEQ ID NO:57,
comprising a LCDR1 shown in SEQ ID NO:58,
comprising a LCDR2 shown in SEQ ID NO:59, and
comprising a LCDR3 shown in SEQ ID NO:60; or
5) comprising a HCDR1 shown in SEQ ID NO:61,
comprising a HCDR2 shown in SEQ ID NO:62,
comprising a HCDR3 shown in SEQ ID NO:63,
comprising a LCDR1 shown in SEQ ID NO:64,
comprising a LCDR2 shown in SEQ ID NO:65, and
comprising a LCDR3 shown in SEQ ID NO:66; or
6) comprising a HCDR1 shown in SEQ ID NO:67,
comprising a HCDR2 shown in SEQ ID NO:68,
comprising a HCDR3 shown in SEQ ID NO:69,
comprising a LCDR1 shown in SEQ ID NO:70,
comprising a LCDR2 shown in SEQ ID NO:71, and
comprising a LCDR3 shown in SEQ ID NO:72; or
7) comprising a HCDR1 shown in SEQ ID NO:73,
comprising a HCDR2 shown in SEQ ID NO:74,
comprising a HCDR3 shown in SEQ ID NO:75,
comprising a LCDR1 shown in SEQ ID NO:76,
comprising a LCDR2 shown in SEQ ID NO:77, and
comprising a LCDR3 shown in SEQ ID NO:78; or
8) comprising a HCDR1 shown in SEQ ID NO:79,
comprising a HCDR2 shown in SEQ ID NO:80,
comprising a HCDR3 shown in SEQ ID NO:81,
comprising a LCDR1 shown in SEQ ID NO:82,
comprising a LCDR2 shown in SEQ ID NO:83, and
comprising a LCDR3 shown in SEQ ID NO:84; or
9) comprising a HCDR1 shown in SEQ ID NO:85,
comprising a HCDR2 shown in SEQ ID NO:86,
comprising a HCDR3 shown in SEQ ID NO:87,
comprising a LCDR1 shown in SEQ ID NO:88,
comprising a LCDR2 shown in SEQ ID NO:89, and
comprising a LCDR3 shown in SEQ ID NO:90; or
10) comprising a HCDR1 shown in SEQ ID NO:73,
comprising a HCDR2 shown in SEQ ID NO:74,
comprising a HCDR3 shown in SEQ ID NO:75,
comprising a LCDR1 shown in SEQ ID NO:109,
comprising a LCDR2 shown in SEQ ID NO:77, and
comprising a LCDR3 shown in SEQ ID NO:78; or
11) comprising a HCDR1 shown in SEQ ID NO:73,
comprising a HCDR2 shown in SEQ ID NO:74,
comprising a HCDR3 shown in SEQ ID NO:75,
comprising a LCDR1 shown in SEQ ID NO:110,
comprising a LCDR2 shown in SEQ ID NO:77, and
comprising a LCDR3 shown in SEQ ID NO:78; or
12) comprising a HCDR1 shown in SEQ ID NO:73,
comprising a HCDR2 shown in SEQ ID NO:74,
comprising a HCDR3 shown in SEQ ID NO:75,
comprising a LCDR1 shown in SEQ ID NO:111,
comprising a LCDR2 shown in SEQ ID NO:77, and
comprising a LCDR3 shown in SEQ ID NO:78; or
13) comprising a HCDR1 shown in SEQ ID NO:73,
comprising a HCDR2 shown in SEQ ID NO:74,
comprising a HCDR3 shown in SEQ ID NO:75,
comprising a LCDR1 shown in SEQ ID NO:112,
comprising a LCDR2 shown in SEQ ID NO:77, and
comprising a LCDR3 shown in SEQ ID NO:78; or
14) comprising a HCDR1 shown in SEQ ID NO:73,
comprising a HCDR2 shown in SEQ ID NO:74,
comprising a HCDR3 shown in SEQ ID NO:75,
comprising a LCDR1 shown in SEQ ID NO:113,
comprising a LCDR2 shown in SEQ ID NO:77, and
comprising a LCDR3 shown in SEQ ID NO:78; or
15) comprising a HCDR1 shown in SEQ ID NO:73,
comprising a HCDR2 shown in SEQ ID NO:74,
comprising a HCDR3 shown in SEQ ID NO:75,
comprising a LCDR1 shown in SEQ ID NO:114,
comprising a LCDR2 shown in SEQ ID NO:77, and
comprising a LCDR3 shown in SEQ ID NO:78; or
16) comprising a HCDR1 shown in SEQ ID NO:73,
comprising a HCDR2 shown in SEQ ID NO:74,
comprising a HCDR3 shown in SEQ ID NO:75,
comprising a LCDR1 shown in SEQ ID NO:115,
comprising a LCDR2 shown in SEQ ID NO:77, and
comprising a LCDR3 shown in SEQ ID NO:78; or
17) comprising a HCDR1 shown in SEQ ID NO:73,
comprising a HCDR2 shown in SEQ ID NO:74,
comprising a HCDR3 shown in SEQ ID NO:75,
comprising a LCDR1 shown in SEQ ID NO:116,
comprising a LCDR2 shown in SEQ ID NO:77, and
comprising a LCDR3 shown in SEQ ID NO:78.

7. Antibody or antigen-binding fragment thereof, which specifically binds to respiratory syncytial virus fusion protein (RSV-F) and/or neutralizes respiratory syncytial virus, the antibody or antigen-binding fragment thereof comprises:
a heavy chain variable region, the heavy chain variable region has at least 80% identity with any one amino acid sequence selected from the group consisting of the following amino acid sequences: SEQ ID NO: 1, 3, 5, 7, 9, 11, 13, 15, 17, 19, 21, 23, 25 and 27; preferably, the heavy chain variable region has CDRs identical to that of the amino acid sequence of SEQ ID NO: 1, 3, 5, 7, 9, 11, 13, 15, 17, 19, 21, 23, 25 or 27, and the non-CDRs of the heavy chain variable region have a sequence with at least 80% identity with the non-CDRs of the amino acid sequence: SEQ ID NO: 1, 3, 5, 7, 9, 11, 13, 15, 17, 19, 21, 23, 25 or 27; preferably, the heavy chain variable region has any one amino acid sequence selected from the group consisting of the following amino acid sequences: SEQ ID NO: 1, 3, 5, 7, 9, 11, 13, 15, 17, 19, 21, 23, 25 and 27; and/or
a light chain variable region, the light chain variable region has at least 80% identity with any one amino acid sequence selected from the group consisting of the following amino acid sequences: SEQ ID NO: 2, 4, 6, 8, 10 , 12, 14, 16, 18, 20, 22, 24, 26, 28, 29, 30, 31, 32, 33, 34, 35 and 36; preferably, the light chain variable region has CDRs identical to that of the amino acid sequence of SEQ ID NO: 2, 4, 6, 8, 10, 12, 14, 16, 18, 20, 22, 24, 26, 28, 29, 30, 31, 32, 33, 34, 35 or 36, and the non-CDRs of the light chain variable region have a sequence with at least 80% identity with the non-CDRs of SEQ ID NO: 2, 4, 6, 8, 10, 12, 14, 16, 18, 20, 22, 24, 26, 28, 29, 30, 31, 32, 33, 34, 35 or 36; preferably, the light chain variable region has any one amino acid sequence selected from the group consisting of the following amino acid sequences: SEQ ID NO: 2, 4, 6, 8, 10, 12, 14, 16, 18, 20, 22, 24, 26, 28, 29, 30, 31, 32, 33, 34, 35 and 36.

8. The antibody or antigen-binding fragment thereof according to claim 7, comprising a heavy chain variable region and a light chain variable region, wherein the amino acid sequence pair of the heavy chain variable region/light chain variable region is selected from the group consisting of the following amino acid sequence pairs: SEQ ID NO: 1/2, 3/4, 5/6, 7/8, 9/10, 11/12, 13/14, 15/16, 17/18, 19/20, 21/22, 23/24, 25/26, 27/28, 21/29, 21/30, 21/31, 21/32, 21/33, 21/34, 21/35 and 21/36;
preferably, the amino acid sequence pair of the heavy chain variable region/light chain variable region is selected from the group consisting of the following amino acid sequence pairs: SEQ ID NO: 13/14,19/20, 21/22, 23/24, 25/26, 27/28, 21/29, 21/30, 21/31, 21/32, 21/33, 21/34, 21/35 and 21/36.

9. The antibody or antigen-binding fragment thereof according to any one of claims 2-8, comprising a heavy chain constant region and/or a light chain constant region, preferably comprising a murine or humanized heavy chain constant region and/or light chain constant region; preferably, the amino acid sequence of the humanized heavy chain constant region is as shown in SEQ ID NO: 117, and the amino acid sequence of the humanized light chain constant region is as shown in SEQ ID NO:118.

10. The antibody or antigen-binding fragment thereof according to claim 9, the amino acid sequence pair of its heavy chain/light chain is selected from the group consisting of the following amino acid sequence pairs: SEQ ID NO:119/120, 121/122 and 123/124.

11. The antibody or antigen-binding fragment thereof according to any one of claims 2-10, wherein each CDR is defined according to Kabat definition, Chothia definition, Abm definition and/or Contact definition.

12. The antibody or antigen-binding fragments thereof according to claim 11, wherein each CDR is defined according to Kabat definition or Chothia definition.

13. a nucleic acid molecule, the nucleic acid molecule has a nucleotide sequence encoding the antibody or antigen-binding fragment thereof according to any one of claims 2-12.

14. a expression vector, the expression vectors comprises the nucleic acid molecule according to claim 13.

15. a host cell, the host cell comprises the expression vector according to claim 14.

16. A method of producing an antibody or antigen-binding fragment thereof, comprising culturing the host cells according to claim 15, and recovering the antibody or antigen-binding fragment thereof expressed by the method from the culture.

17. The method according to claim 16, wherein the host cell is a prokaryotic cell or a eukaryotic cell.

18. The method according to claim 16 or 17, wherein the host cell is an E. coli cell, a yeast cell, an insect cell, a plant cell or a mammalian cell.

19. The method according to claim 16, wherein the host cell is a Chinese hamster ovary cell (CHO), CHO cell variant, 293 cell or NSO cell.

20. A pharmaceutical composition, comprising at least one antibody or antigen-binding fragment thereof according to any one of claims 1-12, and pharmaceutically acceptable excipients.

21. Use of the antibody or antigen-binding fragment thereof according to any one of claims 1-12 or the pharmaceutical composition according to claim 20 in the preparation of a medicament for the treatment and/or prevention of respiratory syncytial virus (RSV) infection or symptoms related to RSV infection.

22. The antibody or antigen-binding fragment thereof according to any one of claims 1-12 or the pharmaceutical composition according to claim 20, for use in the treatment and/or prevention of respiratory syncytial virus (RSV) infection or symptoms related to RSV infection.

23. A method for treating and/or preventing respiratory syncytial virus (RSV) infection or symptoms related to RSV infection, comprising administering a therapeutically effective amount of the antibody or antigen-binding fragment thereof according to any one of claims 1-12 or the pharmaceutical composition according to claim 20 to a subject in need thereof.

24. Use of an active ingredient, the active ingredient is selected from the group below: the antibody or antigen-binding fragment thereof according to any one of claims 1-12, wherein, the active ingredient is used in the preparation of a detection plate or kit, the detection plate or kit is used for the detection of respiratory syncytial virus (RSV).

25. A detection plate, comprising: a substrate and a test strip, wherein the test strip contains the antibody or antigen-binding fragment thereof according to any one of claims 1-12.

26. A kit, comprising:
(1) a first container, the first container containing the antibody or antigen-binding fragment thereof according to any one of claims 1-12; and / or
(2) a second container, the second container containing a secondary antibody against the antibody according to any one of claims 1-12;
or, the kit contains the detection plate according to claim 25.
